# EUROPEAN PATENT SPECIFICATION

(11) **EP 1 896 488 B1**
(45) Date of publication and mention of the grant of the patent: **19.05.2010**
(21) Application number: 07753283.6
(22) Date of filing: 15.03.2007
(51) Int. Cl.: C07D 498/18

(54) **PROCESS FOR PURIFYING TACROLIMUS**
VERFAHREN ZUR REINIGUNG VON TACROLIMUS
PROCEDE DE PURIFICATION DE TACROLIMUS

(30) Priority: 15.03.2006 US 782753 P; 25.09.2006 US 847323 P; 20.11.2006 US 860359 P; 13.12.2006 US 874823 P; 10.01.2007 US 879869 P
(43) Date of publication of application: 12.03.2008
(73) Proprietor: TEVA Gyógyszergyár Zártkörüen Müködö Részvénytársaság, Pallagi ut 13 4042 Debrecen (HU)
(72) Inventor: CVAK, Ladislav, 746 01 Opava-Zlatniky (CZ); BUCHTA, Martin, 747 14 Ludgerovice 601 (CZ); JEGOROV, Alexandr, 373 16 Dobra Voda (CZ); BLATNY, Pavel, 746 01 Opava (CZ); KERI, Vilmos, H-4028 Debrecen (HU); CSORVASI, Andrea, H-4027 Debrecen (HU); SIMON, Angela, H-4027 Debrecen (HU); MAKO, Gyorgyne, H-4031 Debrecen (HU)
(74) Representative: Wong, Kit Yee
(86) International application number: PCT/US2007/006642
(87) International publication number: WO 2007/106587

(56) References cited:
- WO-A-2005/098011
- WO-A-2007/013017
- US-B1- 6 492 513
- US-B1- 6 576 135

## Description

### FIELD OF INVENTION

The present invention relates to a process for the purification of Tacrolimus.

### BACKGROUND OF THE INVENTION

Tacrolimus, [3*S*-[3*R*[*E*(1*S*, 3*S*, 4*S*)], 4*S*, 5*R*, 8*S*, 9*E*, 12*R*, 14*R*, 15*S*, 16*R*, 18*S*, 19*S*, 26a*R*]]-5,6,8,11,12,13,14,15,16,17,18,19,24,25,26,26a-hexadecahydro-5,19-dihydroxy-3-[2-(4-hydroxy-3-methoxycyclohexyl)-1-methylethenyl]-14,16-dimethoxy-4,10,12,18-tetramethyl-8-(2-propenyl)-15,19-epoxy-3H-pyrido[2,1-*c*][1,4]oxaazacyclotricosine-1,7,20,21(4H,23H)-tetrone, monohydrate (previously known as FK506), has a molecular weight of 822.05, the formula C₄₄N₆₉NO₁₂H₂O, and the structural formula

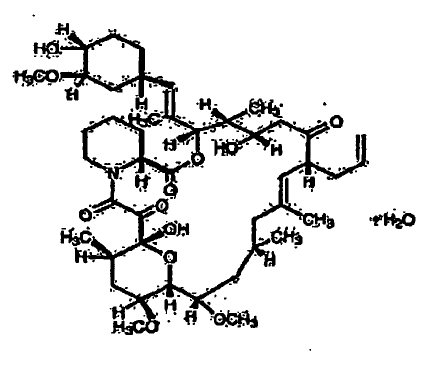

Tacrolimus is a naturally occurring macrolide immunosuppressant produced by *Streptomyces tsukubaensis*. It is marketed under the name PROGRAF® by Fujisawa, and is available for oral administration as capsules (Tacrolimus capsules). Tacrolimus prolongs the survival of the host and transplanted graft in animal transplant models of liver, kidney, heart, bone marrow, small bowel and pancreas, lung and trachea, skin, cornea, and limb by inhibiting T-lymphocyte activation. In animals, Tacrolimus has been demonstrated to suppress some humoral immunity and, to a greater extent, cell-mediated reactions, such as allograft rejection, delayed type hypersensitivity, collagen-induced arthritis, experimental allergic encephalomyelitis, and graft versus host disease.

Tacrolimus was first described in U.S. Patent No. 4,894,366 and in European Patent No. EP 0 184 162. When preparing Tacrolimus, two analogues of Tacrolimus, which are impurities, are also obtained: Ascomycin, which has the structural formula 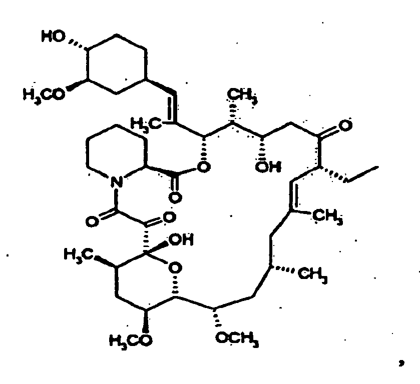 and Dihydrotacrolimus, which has the structural formula.

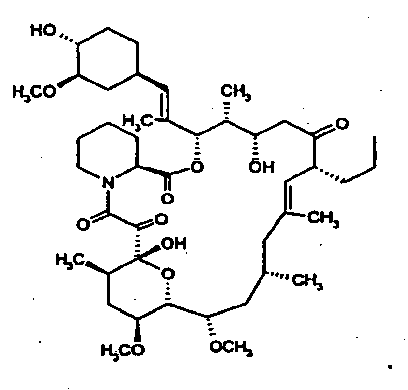

Separation of Tacrolimus from these impurities by conventional methods, such as crystallization, is difficult because of the structural similarity. Therefore, purification by column chromatography is required. The efficiency of the purification can be improved using silver ions in the column chromatography. The silver ions interact with the double bond of the allyl side chain that is present in Tacrolimus, but is absent in Ascomycin and Dihydrotacrolimus in which the side chain is an alkyl group. In Ascomycin, the side chain is an ethyl group, and, in Dihydrotacrolimus, the side chain is a propyl group. As a result, the bond between the Tacrolimus and the stationary phase or to the mobile phase is strengthened, and, thus, the difference in the retention time of Tacrolimus compared to the retention time of its analogues is increased

U.S. Patent No. 6,492,513 discloses separating Tacrolimus from the impurities using a sulfonic acid group-containing strong cation exchange resin pretreated with silver ions, and an eluent of acetone or ethyl acetate and methanol. As Tacrolimus is sensitive to the disclosed exchange resin, contamination of the Tacrolimus would be expected.

U.S. Patents Nos. 6,576,135 and 6,881,341 disclose a two-step column chromatography process for the separation of Tacrolimus from the impurities, particularly tacrolimus-demethyl analogue. One disclosed step involves adsorbing a mixture containing Tacrolimus to a nonionic adsorption resin, and eluting with an aqueous solvent containing silver ions. The other disclosed step involves adsorbing the mixture to a basic active alumina and eluting with an organic solvent to perform the separation. The use of water in the eluent results in the formation of isomers of Tacrolimus, thereby contaminating the Tacrolimus. In addition, the use of an eluent containing free silver ions requires the separation of the silver ions from the eluent from the Tacrolimus following elution from the column.

International Patent Application Publication No. WO 05/054253 discloses the chromatographic separation of Tacrolimus using silica gel, optionally, either in reverse phase chromatography or pretreated with silver, following treatment with ammonia gas to phase out impurities. The disclosed eluent was a mixture of n-butanol, acetonitrile, and a buffer for the reverse phase separation, and a mixture of ethylacetate and hexane for the silver treated silica gel separation.

International Patent Application Publication No. WO 05/098011 discloses a separation of Tacrolimus from impurities by silica gel chromatography, where the silica gel is optionally pretreated with silver. Optionally, the Tacrolimus is further purified by reverse phase chromatography using untreated silica. A C-8 column is exemplified for the reverse phase chromatography. Exemplified eluents include ethyl acetate in hexane for the silver treated column and a mixture of acetonitrile, n-butanol, and buffer for the reverse phase separation.

International Patent Application Publication No. WO 05/010015 discloses the separation using a non-ionic adsorption resin without silver and with an eluent containing THF or acetonitrile, water, and, optionally, an additional organic solvent.

There is a need in the art for a method for purifying Tacrolimus, particularly from Ascomycin and Dihydrotacrolimus that would be suitable for use on industrial scale. The present invention provides such a method.

### SUMMARY OF THE INVENTION

In one embodiment the present invention provides a process for separating Tacrolimus from impurities comprising: a) loading a mixture comprised of tacrolimus and impurities in a bed of sorbent pretreated with silver ions, wherein the sorbent is cyano silica-gel; and b) eluting the mixture from the bed of sorbent resin to separate Tacrilomus from impurities present in the mixture.

### BRIEF DESCRIPTION OF THE DRAWINGS

Figure 1 shows HPLC analysis of the chromatographic fractions (mg/l) according to example 2.
Figure 2 shows HPLC analysis of the chromatographic fractions (mg/l) according to example 5.
Figure 3 shows HPLC analysis of the chromatographic fractions (mg/l) according to example 21.
Figure 4 shows HPLC analysis of the chromatographic fractions (mg/l) according to example 25.
Figure 5 shows HPLC analysis of the chromatographic fractions (mg/ml) according to comparative example 23.

### DETAILED DESCRIPTION OF THE INVENTION

The present invention is directed to a process for the chromatographic purification of Tacrolimus using a sorbent pretreated with silver ions for the stationary phase ("silver modified sorbent"), where the sorbent is cyano silica-gel.
The process of the invention is particularly useful for the separation of Tacrolimus from Ascomycin and Dibydratacrolimus.

The process of the present invention is suitable for industrial use. It can be carried out with an eluent that does not contain water and/or silver ions, and provides a more efficient separation of Tacrolimus from Dihydrotacrolimus and Ascomycin. An eluent that does not contain water avoids contamination of the Tacrolimus by facilitating the formation of its isomers. Also, by avoiding use of silver ions in the eluent, the contamination of the final product with silver ions is substantially reduced. The use of a strong acidic resin, to which Tacrolimus is sensitive, and utilization of two-step chromatography, can also be avoided in the process of the present invention.

The Tacrolimus, which is present in a mixture with impurities, is loaded on the silver modified sorbent. The loading can be done, for example, by dissolving the mixture in a solvent, preferably a minimal amount of solvent, that allows dissolution, and loading the solution on the silver modified sorbent as a concentrated solution. A minimal amount of solvent can be about 2 times of volume solvent mixture calculated for the starting tacrolimus mass. The solvent in which the mixture is dissolved in can be the same as that used during elution. Examples of such solvents are provided below. The mixture can also be loaded in other manners, including as oil or as a solid residue obtained from evaporation of a non-concentrated solution of the mixture.

The mixture comprised of Tacrolimus is then eluted with a solvent or solvents from the silver modified sorbent. Preferably the solvent is substantially anhydrous. In one embodiment, a non-aqueous eluent is used containing either a polar organic solvent selected from the group consisting of C₃₋₉ linear or branched ketone, C₃₋₇ linear or branched ester, C₁₋₇ linear or branched alcohol, C₂₋₈ linear, branched or cyclic ether, C₂₋₅ nitrile, and mixtures thereof, or a mixture of a polar organic solvent and an a-polar organic solvent selected from the group consisting of C₅₋₈ linear, branched or cyclic hydrocarbon, C₆₋₁₀ aromatic hydrocarbon, and mixtures thereof. The eluents are preferably anhydrous, i.e., water free, or have a water content that is sufficiently low to prevent the formation of isomers of Tacrolimus. As used herein, the term "anhydrous" refers to eluents that are free of water, except, possibly, as a trace impurity that is insufficient to result in the formation of a detectable amount of isomers of Tacrolimus. Preferably, the eluent contains less than 2% by weight, more preferably, less than 1% by weight, most preferably, less than 0.05% by weight of water.

Preferably, the C₃₋₉ linear or branched ketone is acetone, ethylmethyl ketone, or isobutylmethyl ketone. Preferred C₃₋₇ linear or branched esters include ethylacetate, n-propylacetate, isopropylacetate, n-butylacetate, and ethylpropionate. Preferred C₁₋₇ linear or branched alcohols include methanol, ethanol, isopropanol, n-propanol, n-butanol, and isobutanol. Preferred C₂₋₈ linear, branched, or cyclic ethers include tert-butylmethyl ether, diethylether, diisopropylether, and tetrahydrofuran. A preferred C₂₋₅ nitrile is acetonitrile. Preferably, the C₅₋₈ linear, branched, or cyclic hydrocarbon is hexane, heptane, octane, cyclohexane, or cycloheptane. Preferably, the C₆₋₁₀ aromatic hydrocarbon is toluene. The preferred polar organic solvent is acetone. Preferably, the eluent contains acetone as a single solvent or in a mixture with another solvent, and, more preferably, the eluent contains acetone and hexane or ethylacetate.

When a mixture of acetone and hexane is used, at least one of the following solvent ratios: about 1:1, about 2:3, about 1:4 or about 1:9, respectively, may be used, depending on the sorbent that is used. When a mixture of ethylacetate and acetone is used, the solvent ratio is, preferably, about 50-80% acetone by volume, more preferably, about 30:70 ethyl acetate to acetone by volume. Optionally, elution of the Tacrolimus comprises a gradient elution process, starting with elution with a mixture of solvents, preferably, of acetone and hexane, preferably in a ratio of about 3:2, followed by elution with about 100 percent of a single solvent, preferably, acetone.

Preferably, the type of elution process is chosen according to the length of the column. Preferably, with a short column, for example a column of about 10 cm length, gradient elution is preferred, and, with a longer column, for example a column of about 1 m length, gradient elution is not used.

In a preferred process of the invention, a column is packed with a silver modified sorbent, which is then washed with eluent. A solution of Tacrolimus in the eluent is then loaded, followed by elution of Tacrolimus, Ascomycin, and Dihydrotacrolimus from the column, and recovery and analysis of the obtained samples. The eluent is preferably anhydrous.

Tacrolimus of any purity can be purified with the process of the invention, and be the crude Tacrolimus. Tacrolimus purified with the process of the invention may contain at least one of the following impurities: Ascomycin, typically in an amount of about 0 to about 10 percent area by HPLC of, and/or Dihydrotacrolimus, typically in an amount of about 6 percent to about 0 percent area by HPLC. Crude Tacrolimus containing about 7.9 percent area by HPLC of Ascomycin and/or about 3.8 percent area by HPLC of Dihydrotacrolimus has been purified with the process of the invention.

The order of elution is dependent on the nature of the sorbent and the eluent. Typically, Ascomycin and Dihydrotacrolimus are eluted before Tacrolimus, as the Tacrolimus, has a greater affinity to the silver modified sorbent.

The cyano-silica gel used for the preparation of silver modified cyano-silica gel can be a commercial sorbent or it can be prepared by treatment of silica gel with trichloro-3-cyanopropyl silane, resulting in silica gel covalently bound on its surface to cyanoalkyl group.

Preferably, for efficient purification, the sorbent has a specific surface area of at least about 50 m²/g, more preferably, of about 50 to about 600 m²/g.

Preferably, the silver modified sorbent is prepared by mixing the sorbent, a source of silver cations, and a solvent selected from the group consisting of a C₁₋₄ alcohol, water, and a mixture of water and a water miscible solvent, and drying the resulting mixture. Preferably, when the solvent is a C₁₋₄ alcohol, the silver cation source and the C₁₋₄ alcohol are combined initially to obtain a solution, and the sorbent is then added to obtain a suspension. The resulting suspension is then dried, preferably, in a rotary evaporator, followed by drying in a vacuum oven for about 15 minutes to about 5 hours.

The C₁₋₄ alcohol preferably comprises a single alcohol or a mixture of alcohols. The preferred C₁₋₄ alcohol is methanol. Preferably, when the solvent is a C₁₋₄ alcohol, the source of silver ion and the C₁₋₄ alcohol are heated to about the reflux temperature of the solvent, to obtain a solution.

Inorganic silver salt or inorganic silver complex salt can be used. The salt or the complex can be contacted with the sorbent as a solution. Preferred silver salts include silver acetate, silver sulphate, and silver nitrite. Preferred silver complexes include a silver amino complex, a silver cyano complex, and a silver thiosulfate complex, and silver acetate. More preferably, the silver source is silver nitrate,

The sorbent can comprise of different amount of silver ions. The silver modified sorbent obtained after drying preferably contains about 0.1 percent to about 15 percent w/w of silver salt, and, more preferably, about 3 percent to about 13 percent w/w of silver salt.

Preferably, the eluted fractions are recovered by collecting groups of fractions, and evaporating the solvent, thus, providing a sample. The recovered sample is then analyzed, preferably, by HPLC, providing the content of Tacrolimus, Ascomycin, and Dihydrotacrolimus in the sample. Typically, as the elution process progresses, the level of Ascomycin and Dihydrotacrolimus decreases while the level of Tacrolimus increases. Preferably, the process is repeated, so that the final sample contains substantially pure Tacrolimus. Preferably, the final sample contains at least about 93 percent area by HPLC of Tacrolimus, more preferably, at least about 95 percent area by HPLC of Tacrolimus, and, even more preferably, at least about 99 percent area by HPLC. Preferably, the final sample also contains less than about 0.20 percent area by HPLC of Ascomycin, more preferably, less than about 0.09 percent area by HPLC, and, even more preferably, less than about 0.06 percent area by HPLC. Preferably, the finial sample also contains less then about 0.04 percent area by HPLC of Dihydrotacrolimus, and, more preferably, less than about 0.03 percent area by HPLC of Dihydrotacrolimus.

The column may be regenerated and used for an additional purification process.

The eluted Tacrolimus may contain silver ions that come off the resin; the amount of such silver ions is substantially less than if an eluent containing silver ions is used. The eluted Tacrolimus can be separated from the silver ions by evaporating the eluent to obtain a residue; dissolving the residue in an organic solvent; admixing with a solution comprising a reagent that precipitates silver ions; filtering the precipitated silver salt. The reagent can be a chloride salt, such as sodium or ammonium chloride, whose addition would result in precipitation of silver chloride.
Another method for removal the silver ions is passing the eluate through a column filled with sodium chloride modified silica gel. Sodium chloride modified silica gel can be easily prepared by mixing of silica gel with aqueous solution of sodium chloride.

Typically, the organic solvent is a solvent in which silver salts are immiscible. Preferably, the organic solvent is selected from a group consisting of: ethyl acetate, toluene, normal or isopropyl acetate n-butyl acetate, isobutyl acetate, acetone, ethyl methyl ketone, isobutyl methyl ketone, ethanol, propanol, n-butanol, iso-butanol, mixtures of ethylacetate and acetone, mixtures of toluene and acetone, and mixture thereof with water. Preferably, the solvent is ethylacetate.

Preferably, the organic solvent is present in an amount of about 2 volumes per gram of residue to about 30 volumes per gram of the residue, more preferably, of about 10 to about 30.

Typically, reagents that precipitate silver ions form a silver salt having a low solubility in water and in organic solvents. Preferably, the reagent includes a counter ion selected from a group consisting of acetate, sulfate, nitrite, bromate, salicylate, iodate, chromate, carbonate, citrate, phosphate, chloride, stearate, sulfide, bromide, iodide, cyanide, benzoate, oxalate, sulfite, and thiocyanate. More preferably, the counter ion is carbonate, citrate, phosphate, chloride, stearate, sulfide, bromide, iodide, cyanide, benzoate, oxalate, sulfite, or thiocyanate. Most preferably, the counter ion is bromide, iodide, stearate, sulfide, or cyanide. Preferably, the precipitating reagent is a salt comprising the counter ion. Most preferably, the precipitating reagent is NH₄Cl.

Preferably, the precipitating reagent is added in an amount of 1 to about 5 mole equivalents per mole equivalent of silver, and, more preferably, in an amount of about 1.2 to about 4 mole equivalents per mole equivalent of silver. Preferably, the solution of the reagent precipitating silver ions is an aqueous solution. Preferably, the aqueous solution is added to the solution of the residue in the organic solvent. The addition of the aqueous solution comprising a reagent for precipitating silver provides a system having at least two phases, including an organic phase, comprising the organic solvent, an aqueous phase, and a precipitate of the silver salt. Preferably, the silver salt is silver chloride. The salt is then filtered, and the filtrate is subjected to phase separation, providing an organic phase comprising the eluted Tacrolimus, substantially free of silver ions. Typically, the term "free of", as used herein in reference to Tacrolimus refers to Tacrolimus containing less than 20 ppm of silver ions. Preferably, the Tacrolimus free of silver ions contains less than 10 ppm of silver ions. The level of silver ions can be determined by any method known to a skilled artisan, for example, by the method described in the US Pharmacopeias.

The Tacrolimus obtained directly from the column or following separation of silver ions may be further purified by a crystallization process, preferably comprising dissolving the final sample in a mixture of 2-propanol and n-heptane, followed by filtering. The filtrate is then preferably combined with a mixture of water and n-heptane, followed by cooling to obtain a precipitate. The precipitate is then preferably recovered by filtration and washing with water and then with n-heptane, followed by drying.

Preferably, prior to recovering the precipitate, the filtrate combined with the mixture of solvents is maintained for about 24 hours.

The further purified sample preferably contains Tacrolimus having a purity higher than that obtained with the chromatographic process. Typically, after crystallization, the levels of any remaining Ascomycin and Dihydrotacrolimus decrease further, and the level of Tacrolimus increases. Preferably, the purified sample contains at least 97 percent area by HPLC of Tacrolimus, more preferably, at least about 99 percent area by HPLC or at least 99.5% and even more preferably, at least 99.9 percent area by HPLC.

Having thus described the invention with reference to particular preferred embodiments and illustrative examples, those in the art can appreciate modifications to the invention as described and illustrated that do not depart from the scope of the invention as disclosed in the specification. The Examples are set forth to aid in understanding the invention but are not intended to, and should not be construed to limit its scope in any way.

### EXAMPLES

### HPLC

**Column:** Waters Symmetry C₁₈ 4.6*150 mm 3.5 µm
**Mobile phase:** A: Weigh 200 ml acetonitrile into a 2000 ml volumetric flask,
   fill flask with distilled water, and then add 100 µl 50 percent CH₃COOH (pH=4.0)
B: Add 100 µl 50%-os CH₃COOH to 2000 ml acetonitrile.

**Gradient Table**

| Time (minutes) | **A** Eluent (V/V %) | **B** Eluent (V/V%) |
|---|---|---|
| 0 | 58 | 42 |
| 25 | 58 | 42 |
| 35 | 52 | 48 |
| 45 | 10 | 90 |
| 46 | 10 | 90 |
| 47 | 58 | 42 |

**Flow:** 2.2 ml/min
**Detection wavelength:** 200 nm
**Injection volume:** 20 µl
**Diluent:** Acetonitrile
**Sample temperature** 20°C
**Column temperature**: 60°C
**Run time:** 47 minutes
**Typical retention time:** Tacrolimus 24.5 min
   Tac.I (Ascomycin) RRt=0.91
   Tac.II. (Dihydro-Tacrolimus) RRt-1.35

### Examples marked with an asterisk (*) are provided by way of reference only

### * Example 1:_Preparation of Silver nitrate modified alumina.

Silver nitrate (10.0 g) was dissolved in hot methanol (500 ml), followed by adding alumina (basic active aluminum oxide 90, 63 - 200 µm, activity II - III, manufactured by Merck. 100 g) to obtain a suspension. The suspension was evaporated to dryness on a rotary evaporator. The residue was dried in vacuum (30 mbar) at 70 °C for 5 hours. The silver content: 10 percent w/w.

### * Example 2: Purification of crude Tacrolimus by silver modified alumina, isocratle elution.

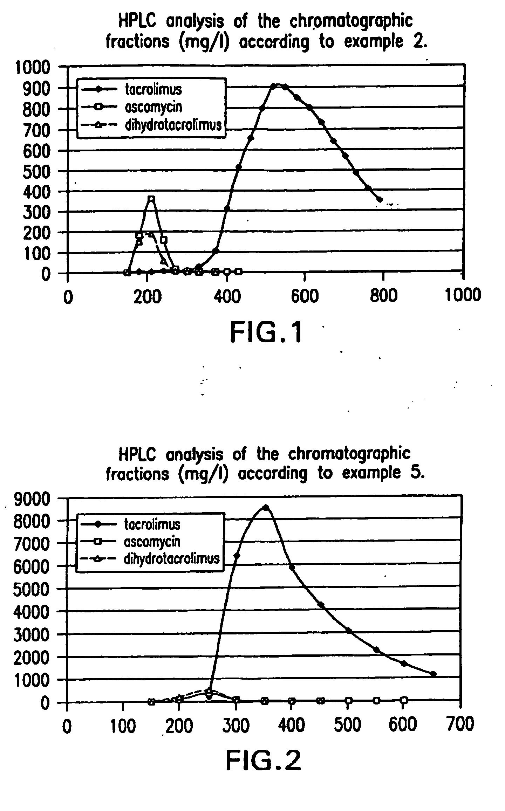

A glass columm (diameter 22 mm, bed height 100 mm, dry filing) was filled with silver nitrate (40 g) modified alumina prepared according to Example 1, and the columm was washed with the mobile phase (about 200 ml), containing 50 percent (v/v) acetone and 50 percent (v/v) n-hexane. Crude Tacrolimus (306 mg) containing, according to HPLC analysis, 79.7 percent Tacrolimus, 7.9 percent Ascomycin and 3.8 percent Dihydrotacrolimus, was dissolved in the mobile phase (10 ml), and the solution was loaded on the column. The column was eluted by the mobile phase (about 20 ml/min), and 30 ml fractions were collected. The factions were analyzed by HPLC, and the reconstructed chromatogram is presented in Figure 1. The first four fractions, representing separated Ascomycin and Dihydrotacrolimus, were concentrated to dryness. The residue (52 mg) contained 2.1 percent Tacrolimus, 31.7 percent Dihydrotacrolimus, and 63.0 percent Ascomycin, according to HPLC analysis. The following three inter-fractions were concentrated to dryness (15 mg), and according to HPLC analysis, they contained 37.5 percent Tacrolimus. The subsequent fractions, containing purified Tacrolimus (15 fractions), were concentrated providing 223 mg of residue that was analyzed by HPLC, and was shown to contain 95.54 percent Tacrolimus, 0.20 percent Ascomycin, and 0.04 percent Dihydrotacrolimus.

### * Example 3: Purification of crude Tacrolimus by silver modified alumina, gradient elution.

The column after example 2 was washed with a mobile phase consisting of 40 percent (v/v) acetone and 60 percent (v/v) n-hexane. Crude Tacrolimus (321 mg, 79.7 percent Tacrolimus, 7.9 percent Ascomycin, and 3.8 percent Dihydrotacrolimus) was dissolved in the mobile phase (10 ml) and then loaded on the column. The elution was carried out with the mobile phase (350 ml) containing 40 percent of acetone and 60 percent of n-hexane (v/v), and the eluate was concentrated to dryness, providing 59 mg of residue, containing, according to HPLC analysis, 3.3 percent Tacrolimus, 34.5 percent Dihydrotacrolimus and 67.0 percent Ascomycin. The column was then eluted with acetone (250 ml), and the eluate was concentrated to provide 218 mg of dry residue, containing 95.7 percent Tacrolimus, 0.09 percent Ascomycin, and 0.03 percent Dihydrotacrolimus.

### * Example 4: Purification of crude Tacrolimus by displacement chromatography of Tacrolimus with modified alumina and acetone eluent

Tacrolimus (3.8 g) was dissolved in acetone (15.6 ml). The solution was passed through a sorbent bed of silver modified alumina (containing about 10 percent silver w/w), having a height of 85 cm that was placed in a glass column, having a diameter of 3.2 cm. The column was then eluted with acetone. The elution rate was 30 ml/hour. One fraction contained 0.3 percent Dihydrotacrolimus, 0.45 percent Ascomycin, and 93.0 percent Tacrolimus.

### * Example 5: Purification of crude Tacrolimus by displacement chromatography of Tacrolimus with modified alumina and acetone eluent

Crude Tacrolimus (2 g) containing, according to HPLC analysis, 1.2 percent Ascomycin and 1.8 percent Dihydrotacrolimus, was dissolved in acetone (20 ml). The solution was passed through a sorbent bed of silver modified alumina (200 g, containing about 10 percent silver w/w). The sorbent bed was then eluted with acetone, and 50 ml fractions were collected and analyzed by HPLC. The reconstructed chromatogram is presented in figure 2. The first three fractions containing macrolides were concentrated, providing 0.41 g of dry residue, containing, according to HPLC analysis, 6.1 percent Ascomycin and 9.4 percent Dihydrotacrolimus. The next seven fractions were concentrated, providing 1.53 g of dry residue, containing, according to HPLC analysis, 0.18 percent of Ascomycin and 0.06 percent of Dihydrotacrolimus.

### * Example 6: Purification of crude Tacrolimus by displacement chromatography of Tacrolimus with modified alumina and acetone:hexane eluent mixture

Tacrolimus (3.8 g) was dissolved in acetone (15.6 ml). The solution was passed through a sorbent bed of silver modified neutral active alumina (containing about 10 percent silver w/w), having a height of 85 cm that was placed in a glass column of 3.2 cm diameter. The sorbent bed was then eluted with acetone and ethylacetate in an acetane:ethylacetate ratio of 70:30. The elution rate was 90 ml/hour. One fraction contained 0.07 percent Dihydrotacrolimus, 0.39 percent Ascomycin, and 94.7 percent Tacrolimus.

### * Example 7: Preparation of silver nitrate modified active acidic alumina

Silver nitrate (75.0 g) was dissolved in hot methanol (1400 ml), followed by the addition of alumina (active acidic aluminum oxide 90, 63 to 200 µm, activity I, manufactured by Merck, 750 g) to obtain a suspension. The suspension was evaporated to dryness on a rotary evaporator. The residue was dried in vacuum. The residue had a silver content of 10 percent w/w.

### * Example 8: Purification of Tacrolimus by chromatography with silver modified active acidic alumina

Tacrolimus (6.0 g) containing, according to EPIC analysis, 1.03 percent Ascomycin and 1.85 percent Dihydrotacrolimus, was dissolved in an acetone and ethylacetate solvent mixture (24 ml) in an acetone:ethylacetate ratio of 70:30. The solution was passed through a sorbent bed of silver modified active acidic alumina (containing about 10 percent silver w/w), having a height of 85 cm that was placed in a glass column of 3.2 cm diameter. The column was eluted at -5°C with acetone and ethyl acetate in an acetone:ethylacetate ratio of 70:30. The elution rate was 90 ml/hour. Fractions were collected and analyzed by HPLC. The chosen main fraction was evaporated and crystallize. According to HPLC analysis, the product contained 96.25 percent Tacrolimus, 0.22 percent Ascomycin, and 0.04 percent Dihydrotacrolimus.

### * Example 9: Crystallization of Tacrolimus

The dry residue obtained by evaporation of the Tacrolimus fractions after chromatography on a silver modified alumina (55.3 g of) was dissolved in a mixture of 2-propanol (150 ml) and n-heptane (150 ml), followed by filtering the solution. The filtrate was then diluted with water (250 ml) and n-heptane (250 ml), and the resulting mixture was cooled and stirred for 24 hours. The precipitated crystalline product was filtered off, washed with water and n-heptane, and dried. 47.8 g of crystalline Tacrolimus was obtained. According to HPLC analysis, the product contained 99.1 percent Tacralimus, 0.33 percent Tacrolimus tautomer II, 0.18 percent Tacrolimus tantomer I, 0.11 percent Ascomycin, and 0.04 percent Dihydrotacrolimus.

### * Example 10: Modification of active alumina

Silver nitrate (20 g) was dissolved in heated methanol (650 ml). Aluminium oxide (750 g) (active neutral, Merck, 0.063-0.200 mm, activity stage I) was added into the solution to obtain a suspension. The suspension was concentrated under reduced pressure till dryness. The silver content of the dried suspension was 2.5 percent w/w.

### * Example 11: Modification of reverse phase Silica Gel

Silver nitrate (20 g) was dissolved in heated methanol (650 ml). Reverse phase Silica Gel (330 g) (LiChroprep RP-18. Merck) was added into the solution to obtain a suspension. The suspension was concentrated under reduced pressure till dryness.

### * Example 12: Modification of adsorption resin

Silver nitrate (30 g) was dissolved in heated methanol (650 ml).
Adsorption resin XAD 1180 (650 g) was added into the solution to obtain a suspension. The suspension was concentrated under reduced pressure till dryness.

### * Examples 13: Modification of adsorption resin

Silver nitrate (30 g) was dissolved in heated methanol (650 ml) Absorption resin SP 207 (650 g) was added into the solution to obtain a suspension. The suspension was concentrated under reduced pressure till dryness.

### * Example 14: Modification of adsorption resin having small particle size

Silver nitrate (30 g) was dissolved in heated methanol (650 ml). Adsorption resin Amberchrom CG 300 M (650 g) was added into the solution to obtain a suspension. The suspension was concentrated under reduced pressure till dryness.

### * Example 15: Modification of adsorption resin having small particle size

Silver nitrate (30 g) was dissolved in methanol (650 ml) heating at reflux temperature. Adsorption resin Amberchrom CG 300 S (650 g) was added into the solution to obtain a suspension. The suspension was concentrated under reduced pressure till dryness.

### * Example 16: Modification of cation exchanger resin

Silver nitrate (30 g) was dissolved in heated methanol (650 ml). Cation exchanger resin SK 104 (650 g) was added into the solution to obtain a suspension. The suspension was concentrated under reduced pressure till dryness.

### * Example 17: Modification of anion exchanger resin

Silver nitrate (30 g) was dissolved in heated methanol (650 ml). Anion exchanger resin IRA 68 (650 g) was added into the solution to obtain a suspension. The suspension was concentrated under reduced pressure till dryness.

### * Example 18: Modification of reverse phase Silica Gel

Silver nitrate (20 g) was dissolved in water (500 ml) at ambient temperature. Concentrated ammonia solution of 40 ml was added to the silver nitrate solution in several portions in presence of reverse phase Silica Gel of 330 g (LiChroprep RP-18, Merck). The solution was concentrated cautiously under high vacuum till dryness.

### * Example 19: Modification of anion exchanger resin

Silver nitrate (30 g) was dissolved in heated methanol (650 ml). Anion exchanger resin IRA 900 (650 g) is added into the solution to obtain a suspension.
The suspension is concentrated under reduced pressure till dryness.

### Example 20: Silver nitrate modified cyano-silica gel

Silver nitrate (10.0 g) was dissolved in hot methanol (500 ml) and cyano-silica gel (Silica gel 100 CN, 15-35 µm, manufactured by Fluka, 100 g) was added into the solution, to obtain a suspension. The suspension was evaporated to dryness on a rotary evaporator. The residue was dried in vacuum (30 mbar) at 70 °C for 5 hours.

### Example 21: Purification of crude Tacrolimus by silver modified cyano-silica gel

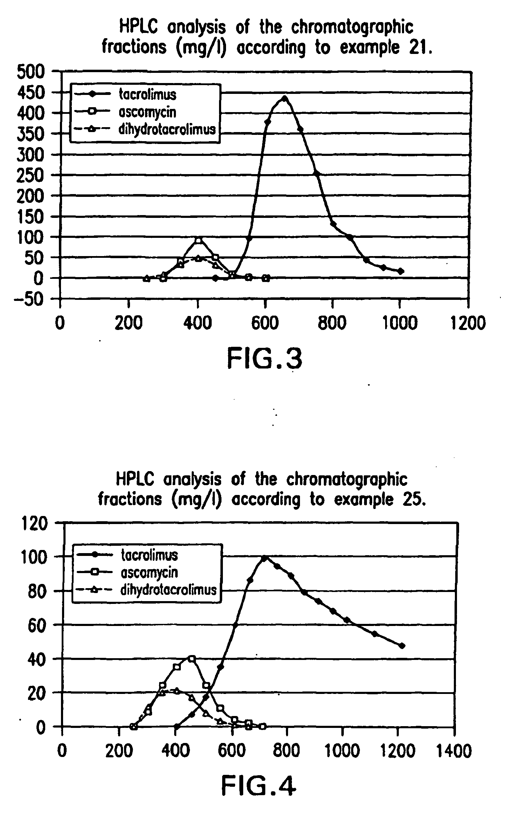

A chromatographic column (diameter 25 mm, 250 mm length) was filled with the sorbent prepared in example 20 (54.4 g), and the column was washed with the mobile phase (mixture acetone and n-hexane 25:75, 1000 ml). Then crude Tacrolimus (510 mg) (79-7 percent Tacrolimus, 7.9 percent Ascomycin and 3.8 percent Dihydrotacrolimus) was dissolved in the mobile phase (25 ml), loaded on the columm, and the column was eluted with the mobile phase, Fractions of 100 ml each were taken and analyzed by HPLC. The reconstructed chromatogram is on Figure 3.

### * Example 22: Silver nitrate modified silica gel

Silver nitrate (10.0 g) was dissolved in hot methanol (500 ml) and silica gel (Silica gel 60 15-40 µm, manufactured by Merck, 100 g) was added into the solution, to obtain a suspension. The suspension was evaporated to dryness on a rotary evaporator. The residue was dried in vacuum (30 mbar) at 70 °C for 8 hours.

### Comparative Example 23: Purification of crude Tacrolimus by silver modified silica gel

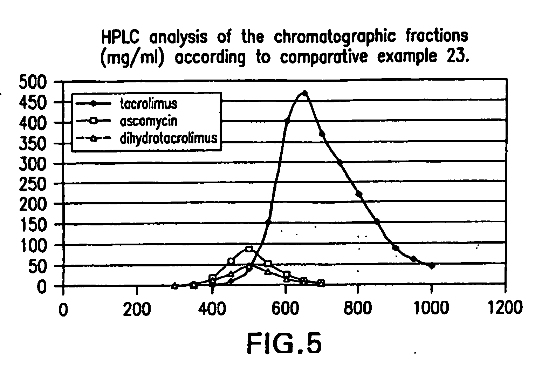

A chromatographic column (diameter 25 mm, 250 mm length) was filled with the sorbent prepared in example 26 (62.6 g), and the column was washed with the mobile phase (mixture acetone and n-hexane 20:80, 1000 ml). Then crude Tacrolimus (500 mg) (79.7 percent Tacrolimus, 7.9 percent Ascomycin and 3.8 percent Dihydrotacrolimus) was dissolved in the mobile phase (25 ml), loaded on the column, and the column was eluted with the mobile phase, Fractions of 100 ml each were taken and analyzed by HPLC. The reconstructed chromatogram is on Figure 5.

### * Example 24: Silver nitrate modified zirconium oxide

Silver nitrate (20.0 g) was dissolved in hot methanol (1000 ml), and zirconium oxide (manufactured by Merck, 200 g) was added into the solution to obtain a suspension. The suspension was evaporated to dryness on a rotary evaporator. The residue was dried in vacuum (30 mbar) at 70 °C for 5 hours.

### * Example 25: purification of crude Tacrolimus by silver modified zirconium oxide

A chromatographic column (diameter 25 mm, 250 mm length) was filled with the sorbent prepared in example 24 (345 g) (the preparation of the sorbent was repeated), and the column was washed with the mobile phase (about 1000 ml) (mixture acetone and n-hexane 10:90). Then, crude Tacrolimus (210 mg) (79.7 percent Tacrolimus, 7.9 percent Ascomycin, and 3.8 percent Dihydrotacrolimus) was dissolved in the mobile phase (25 ml), and was loaded on the column, and the column was eluted with the mobile phase. Fractions of 100 ml each wen collected and analyzed by HPLC. The reconstructed chromatogram is on Figure 4.

### * Example 26: Separating Tacrolimus from silver ions

The chosen main fraction-collected according to examples 6 and 8-was evaporated to dryness under reduced pressure. The residue was dissolved in ethyl acetate (20 times the volume of the residue). NH₄Cl (1.3 times the mass of the residue) was dissolved in water (5 times the volume of the residue), and the salt solution was added to the ethyl acetate solution. After 30 minutes stirring, the precipitated silver chloride was filtered, and phases were separated. The macrolide was crystallized from the ethyl acetate phase.

### * Example 27: Separating Tacrolimus from silver ions

The eluate from the column was found to contain about 80 mg/l of silver. The eluate was passed through a column filled with a silica gel modified with sodium chloride. Silver was completely retained on the column. The Silver content was less than 10 ppm. The modified silica gel was prepared by addition of an aqueous solution of sodium chloride to a silica gel followed by homogenization.

## Claims

1. A process for separating Tacrolimus from impurities comprising:
a) loading a mixture comprised of tacrolimus and impurities in a bed of sorbent pretreated with silver ions, wherein the sorbent is cyano silica-gel; and
b) eluting the mixture from the bed of sorbent resin to separate Tacrolimus from impurities present in the mixture.

2. The process according to claim 1, wherein the mixture is eluted with an anhydrous eluent, wherein the anhydrous eluent preferably has less than about 2% water by volume.

3. The process according to any one of claims 1-2, wherein the impurities are Ascomycin and Dihydrotacrolimus.

4. The process according to any one of claims 1-3, wherein the anhydrous eluent is selected from the group consisting of polar organic solvents and mixtures of polar organic solvents and apolar organic solvents.

5. The process according to claim 4, wherein the polar organic solvent is selected from the group consisting of C₃₋₉ linear or branched ketones, C₃₋₇ linear or branched esters, C₁₋₇ linear or branched alcohols, C₂₋₈ linear, branched or cyclic ethers, C₂₋₅ nitriles, and mixtures thereof.

6. The process according to claim 4, wherein the apolar organic solvent is selected from the group consisting of C₅₋₈ linear, branched or cyclic hydrocarbon, C_{6- 10} aromatic hydrocarbon, and mixtures thereof.

7. The process according to any one of the claims 2-6, wherein the anhydrous eluent is selected from the group consisting of acetone, ethyl-methyl ketone, iso-butyl-methyl ketone, ethyl acetate, n-propyl acetate, isopropyl acetate, n-butylacetate, ethylpropionate, methanol, ethanol, isopropanol, n-propanol, n-butanol, iso-butanol, tert-butylmethylether, diethylether, diisopropyl ether, tetrahydrofuran, acetonitrile, toluene, and linear, branched, or cyclic hexane, heptane, octane, cyclohexane, and cycloheptane.

8. The process according to claim 7, wherein the anhydrous eluent comprises acetone, hexane or ethylacetate.

9. The process according to claim 8, wherein the anhydrous eluent comprises acetone and hexane, wherein the acetone and hexane are present in an acetone to hexane ratio of about 1:1, about 2:3, about 1:4, or about 1:9.

10. The process according to claim 8, wherein the eluent is a mixture of acetone and ethyl acetate, and the acetone and ethyl acetate are present in an about 50-80% acetone by volume compared to ethyl acetate.

11. The process according to claim 2, wherein the anhydrous eluent is acetone.

12. The process according to any one of claims 2-11, wherein the Tacrolimus is eluted with a gradient elution, starting the elution with a mixture of solvents, followed by elution with about 100 percent a single solvent, preferably wherein the gradient elution comprises starting the elution with a mixture comprising acetone and hexane, followed by elution with acetone.

13. The process according to claim 12, wherein the acetone and hexane are present in an acetone to hexane ratio of about 3:2 v/v.

14. The process according to any one of claims 1-13, wherein the mixture is eluted with a gradient elution with a column of about 10 cm or less in length.

15. The process according to any one of claims 1-13, wherein the mixture is eluted without a gradient elution with a column of at least about 1 m in length.

16. The process according to any one of claims 1-15, wherein the process comprises filling a column with the silver modified sorbent, washing the silver modified sorbent with an eluent, loading a solution of Tacrolimus in the eluent onto the column, and eluting Tacrolimus, Ascomycin, and Dihydrotacrolimus from the column.

17. The process according to any one of claims 1-16, wherein at least one of the Ascomycin and Dihydrotacrolimus is eluted from the column before the Tacrolimus.

18. The process according to any one of claims 1-16, wherein the Tacrolimus prior to separation comprises up to about 10 percent area by HPLC of Ascomycin and/or up to about 6 percent area by HPLC of Dihydrotacrolimus.

19. The process according to any one of claims 1-18, wherein the sorbent has a specific surface area of at least about 50 m²/g.

20. The process according to any one of claims 1-19 further comprising preparing the silver modified sorbent in a process comprising mixing the sorbent, a source of silver cations, and a solvent selected from the group consisting of one or more C₁₋₄ alcohols, water, and a mixture of water and a water miscible solvent, and drying,

21. The process according to any one of claims 1-20, wherein the cyano silica gel comprises silica gel covalently bound on its surface to cyanoalkyl group.

22. The process according to claim 20, wherein the solvent is a C₁₋₄ alcohol, and the silver cation source and the C₁₋₄ alcohol are combined to form a solution, and the sorbent is then added to form a suspension, and preferably wherein the C₁₋₄ alcohol is methanol.

23. The process according to claim 21, wherein the solvent is a C₁₋₄ alcohol, and the source of silver ion and the C₁₋₄ alcohol are heated to about the solvent reflux temperature to obtain a solution.

24. The process according to claim 20, wherein the solvent is water or a mixture of water and a water miscible solvent, the sorbent is a reverse-phase silica-gel, the silver ion source is dissolved at ambient temperature, and, prior to drying, a solution of ammonia is added to the solution of solvent and silver cation source.

25. The process according to claim 20, wherein the source of silver cations is a solution of a silver salt or of a silver complex, and preferably wherein the source of silver cations comprises at least one of silver nitrate and silver acetate.

26. The process according to claim 20, wherein the source of silver cations is selected from the group consisting of silver nitrate, silver acetate, silver sulfate, a silver cyano complex, and a silver thiosulfate complex.

27. The process according to claim 25, wherein the source of silver cations comprises a silver cyano complex.

28. The process according to claim 20, wherein the silver modified sorbent, after drying, comprises about 0.1 to about 15 percent w/w of silver salt.

29. The process according to any one of claims 1-28, wherein the separated Tacrolimus comprises at least about 93 percent area by HPLC Tacrolimus.

30. The process according to any one of claims 1-28, wherein the separated Tacrolimus comprises less than about 0.20 percent area by HPLC of Ascomycin.

31. The process according to any one of claims 1-28, wherein the separated Tacrolimus comprises less then about 0.04 percent area by HPLC of Dihydrotacrolimus.

32. The process according to any one of claims 1-28, further comprising separating any silver ions eluted with the Tacrolimus from the Tacrolimus.

33. The process according to any one of claims 1-32, comprising evaporating the eluent to obtain a residue, dissolving the residue in an organic solvent, admixing the resulting solution with a solution comprising a reagent that precipitates silver ions, and filtering the precipitated silver salt, wherein the organic solvent is preferably selected from a group consisting of: ethyl acetate, toluene, normal or isopropyl acetate n-butyl acetate, isobutyl acetate, acetone, ethyl methyl ketone, isobutyl methyl ketone, ethanol, propanol, n-butanol, iso-butanol, mixtures of ethylacetate and acetone, mixtures of toluene and acetone, and mixture thereof with water, and more preferably wherein the organic solvent is ethylacetate.

34. The process according to claim 33, wherein the reagent comprises a counter ion selected from a group consisting of acetate, sulfate, nitrite, bromate, salicylate, iodate, chromate, carbonate, citrate, phosphate, chloride, stearate, sulfide, bromide, iodide, cyanide, benzoate, oxalate, sulfite, and thiocyanate, and preferably wherein the precipitating reagent is NH₄Cl, or sodium chloride.

35. The process according to claim 33, further comprising adding the precipitating reagent in an amount of 1 to about 5 mole equivalents per mole equivalent of silver.

36. The process according to claim 33, wherein the solution comprising a reagent that precipitates silver ions in an aqueous solution.

37. The process according to claim 33, comprising passing the eluate through a column filled with a mixture of silica gel and a precipitating agent

38. The process according to any one of claims 1-37, further comprising collecting at least one fraction of effluent having Tacrolimus.

39. The process according to claim 38, further comprising crystallizing the separated Tacrolimus.

## Patentansprüche

1. Verfahren zum Trennen von Tacrolimus von Unreinheiten, wobei das Verfahren folgende Stufen umfasst:
a) beladen eines Gemisches, welches sich aus Tacrolimus und Verunreinigungen zusammensetzt auf ein Bett aus Sorbtionsmittel, welches mit Silberionen vorbehandelt ist, wobei das Sorbtionsmittel ein Cyano-Silicagel ist, und
b) eluieren des Gemisches von dem Bett aus Sorbtionsharz, um Tacrolimus von in dem Gemisch vorhandenen Verunreinigungen zu trennen.

2. Verfahren gemäß Anspruch 1, wobei das Gemisch mit einem wasserfreien Elutionsmittel eluiert wird, wobei das wasserfreie Elutionsmittel bevorzugt weniger als ungefähr 2 Vol.-% Wasser enthält.

3. Verfahren gemäß einem der Ansprüche 1-2, wobei die Verunreinigungen Ascomycin und Dihydrotacrolimus sind.

4. Verfahren gemäß einem der Ansprüche 1-3, wobei das wasserfreie Elutionsmittel ausgewählt ist aus der Gruppe, bestehend aus polaren organischen Lösungsmitteln und Gemischen von polaren organischen Lösungsmitteln und apolaren organischen Lösungsmitteln.

5. Verfahren gemäß Anspruch 4, wobei das polare organische Lösungsmittel ausgewählt ist aus der Gruppe, bestehend aus C₃₋₉ linearen oder verzweigten Ketonen, C₃₋₇ linearen oder verzweigten Estern, C₁₋₇ linearen oder verzweigten Alkoholen, C₂₋₈ linearen, verzweigten oder zyklischen Ethern, C₂₋₅ Nitrilen und Gemischen davon.

6. Verfahren gemäß Anspruch 4, wobei das apolare organische Lösungsmittel ausgewählt ist aus der Gruppe, bestehend aus C₅₋₈ linearen, verzweigten oder zyklischen Kohlenwasserstoffen, C₆₋₁₀ aromatischen Kohlenwasserstoffen und Gemischen davon.

7. Verfahren gemäß einem der Ansprüche 2-6, wobei das wasserfreie Elutionsmittel ausgewählt ist aus der Gruppe, bestehend aus Aceton, Ethylmethylketon, Isobutylmethylketon, Ethylacetat, n-Propylacetat, n-Butanol, Isobutanol, tert-Butylmethylether, Diethylether, Diisopropylether, Tetrahydrofuran, Acetonitril, Toluen und linearem, verzweigtem oder zyklischen Hexan, Heptan, Octan, Cyclohexan und Cycloheptan.

8. Verfahren gemäß Anspruch 7, wobei das wasserfreie Elutionsmittel Aceton, Hexan oder Ethylacetat umfasst.

9. Verfahren gemäß Anspruch 8, wobei das wasserfreie Elutionsmittel Aceton und Hexan umfasst, wobei das Aceton und das Hexan in einem Verhältnis von Aceton zu Hexan von ungefähr 1:1, ungefähr 2:3, ungefähr 1:4 oder ungefähr 1:9 vorliegen.

10. Verfahren gemäß Anspruch 8, wobei das Elutionsmittel ein Gemisch aus Aceton und Ethylacetat ist und das Aceton und Ethylacetat in ungefähr 50-80 Vol.-% Aceton im Vergleich zu Ethylacetat vorliegen.

11. Verfahren gemäß Anspruch 2, wobei das wasserfreie Elutionsmittel Aceton ist.

12. Verfahren gemäß einem der Ansprüche 2-11, wobei das Tacrolimus mit einer Gradientenelution eluiert wird, wobei die Elution mit einem Gemisch an Lösungsmitteln begonnen wird, gefolgt durch Elution mit ungefähr 100 % eines einzelnen Lösungsmittels, wobei bevorzugt die Gradientenelution das Beginnen der Elution mit einem Gemisch, welches Aceton und Hexan enthält, gefolgt von Elution mit Aceton, umfasst.

13. Verfahren gemäß Anspruch 12, wobei das Aceton und Hexan in einem Verhältnis von Aceton zu Hexan von etwa 3:2 (v/v) vorliegen.

14. Verfahren gemäß einem der Ansprüche 1-13, wobei das Gemisch mit einer Gradientenelution mit einer Säule von ungefähr 10 cm oder weniger Länge eluiert wird.

15. Verfahren gemäß einem der Ansprüche 1-13, wobei das Gemisch mit einer Gradientenelution mit einer Säule von einer Länge von wenigstens ungefähr 1 m eluiert wird.

16. Verfahren gemäß einem der Ansprüche 1-15, wobei das Verfahren folgendes umfasst: Befüllen einer Säule mit dem mit Silber modifizierten Sorbtionsmittel, Waschen des mit Silber modifizierten Sorbtionsmittels mit einem Elutionsmittel, Laden einer Lösung von Tacrolimus in dem Lösungsmittel auf die Säule und Eluieren von Tacrolimus, Ascomycin und Dihydrotacrolimus von der Säule.

17. Verfahren gemäß einem der Ansprüche 1-16, wobei wenigstens eines unter Ascomycin und Dihydrotacrolimus von der Säule vor dem Tacrolimus eluiert wird.

18. Verfahren gemäß einem der Ansprüche 1-16, wobei das Tacrolimus vor der Abtrennung bei HPLC bis ungefähr 10 Flächenprozent Ascomycin und/oder bei HPLC bis ungefähr 6 Flächenprozent Dihydrotacrolimus umfasst.

19. Verfahren gemäß einem der Ansprüche 1-18, wobei das Sorbtionsmittel eine spezifische Oberfläche von wenigstens ungefähr 50 m²/g aufweist.

20. Verfahren gemäß einem der Ansprüche 1-19, welches weiter das Herstellen des mit Silber modifizierten Sorbtionsmittels umfasst, in einem Verfahren welches die folgenden Stufen umfasst: Zusammenmischen des Sorbtionsmittels, einer Quelle von Silberkationen und eines Lösungsmittels, ausgewählt aus der Gruppe, bestehend aus einem oder mehreren C₁₋₄ Alkoholen, Wasser und einem Gemisch aus Wasser und einem mit Wasser mischbaren Lösungsmittel, und Trocknen.

21. Verfahren gemäß einem der Ansprüche 1-20, wobei das Cyano-Silicagel Silicagel umfasst, welches kovalent an seiner Oberfläche an eine Cyanoalkylgruppe gebunden ist.

22. Verfahren gemäß Anspruch 20, wobei das Lösungsmittel ein C₁₋₄ Alkohol ist und die Quelle von Silberkationen und der C₁₋₄ Alkohol unter Bildung einer Lösung zusammengegeben werden und das Sorbtionsmittel dann unter Bildung einer Suspension zugegeben wird und wobei bevorzugt der C₁₋₄ Alkohol Methanol ist.

23. Verfahren gemäß Anspruch 21, wobei das Lösungsmittel ein C₁₋₄ Alkohol ist und die Quelle von Silberionen und der C₁₋₄ Alkohol unter Erhalt einer Lösung bis ungefähr auf die Temperatur des Lösungsmittelrückflusses erhitzt werden.

24. Verfahren gemäß Anspruch 20, wobei das Lösungsmittel Wasser oder ein Gemisch aus Wasser und einem mit Wasser mischbaren Lösungsmittel ist, das Sorbtionsmittel ein Umkehrphasen-Silicagel ist, die Quelle von Silberionen bei Raumtemperatur gelöst wird und vor dem Trocknen eine Ammoniaklösung zu der Lösung aus Lösungsmittel und Quelle von Silberkationen zugegeben wird.

25. Verfahren gemäß Anspruch 20, wobei die Quelle von Silberkationen eine Lösung aus einem Silbersalz oder einem Silberkomplex ist und wobei bevorzugt die Quelle von Silberkationen wenigstens eines unter Silbernitrat und Silberacetat umfasst.

26. Verfahren gemäß Anspruch 20, wobei die Quelle von Silberkationen ausgewählt ist aus der Gruppe, bestehend aus Silbernitrat, Silberacetat, Silbersulfat, einem Silber-Cyano-Komplex und einem Silber-Thiosulfat-Komplex.

27. Verfahren gemäß Anspruch 25, wobei die Quelle von Silberkationen einen Silber-Cyano-Komplex umfasst.

28. Verfahren gemäß Anspruch 20, wobei das mit Silber modifizierte Sorbtionsmittel nach dem Trocknen ungefähr 0,1 bis ungefähr 15 % (w/w) Silbersalz umfasst.

29. Verfahren gemäß einem der Ansprüche 1-28, wobei das abgetrennte Tacrolimus bei HPCL wenigstens ungefähr 93 Flächenprozent Tacrolimus umfasst.

30. Verfahren gemäß einem der Ansprüche 1-28, wobei das abgetrennte Tacrolimus bei HPLC weniger als ungefähr 0,20 Flächenprozent Ascomycin umfasst.

31. Verfahren gemäß einem der Ansprüche 1-28, wobei das abgetrennte Tacrolimus bei HPLC weniger als ungefähr 0,04 Flächenprozent Dihydrotacrolimus umfasst.

32. Verfahren gemäß einem der Ansprüche 1-28, welches weiter das Abtrennen von mit dem Tacrolimus eluierten Silberionen von dem Tacrolimus umfasst.

33. Verfahren gemäß einem der Ansprüche 1-32, welches die folgenden Stufen umfasst: Verdampfen des Elutionsmittels, unter Erhalt eines Rückstandes, Lösen des Rückstandes in einem organischen Lösungsmittel, Zumischen der entstehenden Lösung zu einer Lösung, die ein Silberionen präzipitierendes Reagenz umfasst und Filtern des präzipitierten Silbersalzes, wobei das organische Lösungsmittel bevorzugt ausgewählt ist aus der Gruppe, bestehend aus Ethylacetat, Toluen, normalem oder Isopropylacetat n-Butylacetat, lsobutylacetat, Aceton, Ethylmethylketon, lsobutylmethylketon, Ethanol, Propanol, n-Butanol, iso-Butanol, Gemischen von Ethylacetat und Aceton, Gemischen von Toluen und Aceton und Gemischen davon mit Wasser, und wobei mehr bevorzugt das organische Lösungsmittel Ethylacetat ist.

34. Verfahren gemäß Anspruch 33, wobei das Reagenz ein Gegenion umfasst, ausgewählt aus der Gruppe, bestehend aus Acetat, Sulfat, Nitrit, Bromat, Salicylat, lodat, Chromat, Carbonat, Citrat, Phosphat, Chlorid, Stearat, Sulfid, Bromid, lodid, Cyanid, Benzoat, Oxalat, Sulfit und Thiocyanat, und wobei bevorzugt das Fällungsmittel NH₄Cl oder Natriumchlorid ist.

35. Verfahren gemäß Anspruch 33, welches weiter die Zugabe eines Fällungsmittels in einer Menge von 1 bis ungefähr 5 Moläquivalenten pro Moläquivalenten Silber umfasst.

36. Verfahren gemäß Anspruch 33, wobei die Lösung ein Reagenz umfasst, das Silberionen in einer wässrigen Lösung präzipitiert.

37. Verfahren gemäß Anspruch 33, welches das Durchlaufen des Elutionsmittels durch eine mit einem Gemisch aus Silicagel und einem Fällungsmittel gefüllte Säule umfasst.

38. Verfahren gemäß einem der Ansprüche 1-37, welches weiterhin das Sammeln wenigstens einer Fraktion von Eluat mit Tacrolimus umfasst.

39. Verfahren gemäß Anspruch 38, welches weiter das Kristallisieren des abgetrennten Tacrolimus umfasst.

## Revendications

1. Procédé de séparation de tacrolimus d'impuretés comprenant :
a) le chargement d'un mélange constitué de tacrolimus et d'impuretés sur un lit de sorbant prétraité avec des ions argent, où le sorbant est le cyano gel de silice ; et
b) l'élution du mélange du lit de résine sorbante afin de séparer le tacrolimus d'impuretés présentes dans le mélange.

2. Procédé selon la revendication 1, dans lequel le mélange est élué avec un éluant anhydre, où l'éluant anhydre a de préférence moins d'environ 2 % en volume d'eau.

3. Procédé selon l'une quelconque des revendications 1 et 2, dans lequel les impuretés sont l'ascomycine et le dihydrotacrolimus.

4. Procédé selon l'une quelconque des revendications 1 à 3, dans lequel l'éluant anhydre est choisi dans le groupe comprenant les solvants organiques polaires et les mélanges de solvants organiques polaires et de solvants organiques apolaires.

5. Procédé selon la revendication 4, dans lequel le solvant organique polaire est choisi dans le groupe comprenant les cétones linéaires ou ramifiées en C_{3 à} 9, les esters linéaires ou ramifiés en C_{3 à 7}, les alcools linéaires ou ramifiés en C_{1 à 7}, les éthers linéaires, ramifiés ou cycliques en C_{2 à 8}, les nitriles en C_{2 à 5}, et leurs mélanges.

6. Procédé selon la revendication 4, dans lequel le solvant organique apolaire est choisi dans le groupe comprenant un hydrocarbure linéaire, ramifié ou cyclique en C_{5 à 8}, un hydrocarbure aromatique en C_{6 à 10} et leurs mélanges.

7. Procédé selon l'une quelconque des revendications 2 à 6, dans lequel l'éluant anhydre est choisi dans le groupe comprenant l'acétone, l'éthyl-méthyl cétone, l'iso-butyl-méthyl cétone, l'acétate d'éthyle, l'acétate de n-propyle, l'acétate d'isopropyle, l'acétate de n-butyle, le proprionate d'éthyle, le méthanol, l'éthanol, l'isopropanol, le n-propanol, le n-butanol, l'iso-butanol, le tert-butylméthyléther, le diéthyléther, le diisopropyl éther, le tétrahydrofurane, l'acétonitrile, le toluène et l'hexane, l'heptane, l'octane, le cyclohexane et le cycloheptane linéaire, ramifié ou cyclique.

8. Procédé selon la revendication 7, dans lequel l'éluant anhydre comprend l'acétone, l'hexane ou l'acétate d'éthyle.

9. Procédé selon la revendication 8, dans lequel l'éluant anhydre comprend l'acétone et l'hexane, où l'acétone et l'hexane sont présents selon un rapport acétone-hexane d'environ 1:1, d'environ 2:3, d'environ 1:4 ou d'environ 1:9.

10. Procédé selon la revendication 8, dans lequel l'éluant est un mélange d'acétone et d'acétate d'éthyle, et l'acétone et l'acétate d'éthyle sont présents à environ 50 à 80 % en volume d'acétone par rapport à l'acétate d'éthyle.

11. Procédé selon la revendication 2, dans lequel l'éluant anhydre est l'acétone.

12. Procédé selon l'une quelconque des revendications 2 à 11, dans lequel le tacrolimus est dilué avec un gradient d'élution, en commençant l'élution avec un mélange de solvants, suivi par une élution avec environ 100 pour cent de solvant unique, de préférence où le gradient d'élution comprend le début de l'élution avec un mélange comprenant l'acétone et l'hexane, suivi par une élution avec l'acétone.

13. Procédé selon la revendication 12, dans lequel l'acétone et l'hexane sont présents selon un rapport acétone-hexane d'environ 3:2 v/v.

14. Procédé selon l'une quelconque des revendications 1 à 13, dans lequel le mélange est élué avec un gradient d'élution sur une colonne ayant une longueur d'environ 10 cm ou moins.

15. Procédé selon l'une quelconque des revendications 1 à 13, dans lequel le mélange est élué sans gradient d'élution avec une colonne ayant une longueur d'au moins environ 1 m.

16. Procédé selon l'une quelconque des revendications 1 à 15, dans lequel le procédé comprend le remplissage d'une colonne avec le sorbant modifié par des ions argent, le lavage du sorbant modifié par argent avec un éluant, le chargement d'une solution de tacrolimus dans l'éluant sur la colonne et l'élution de tacrolimus, d'ascomycine et de dihydrotacrolimus de la colonne.

17. Procédé selon l'une quelconque des revendications 1 à 16, dans lequel au moins l'ascomycine ou le dihydrotacrolimus est élué de la colonne avant le tacrolimus.

18. Procédé selon l'une quelconque des revendications 1 à 16, dans lequel le tacrolimus avant séparation représente jusqu'à environ 10 pour cent de surface par HPLC d'ascomycine et/ou jusqu'à environ 6 pour cent de surface par HPLC de dihydrotacrolimus.

19. Procédé selon l'une quelconque des revendications 1 à 18, dans lequel le sorbant a une surface spécifique d'au moins environ 50 m²/g.

20. Procédé selon l'une quelconque des revendications 1 à 19, comprenant en outre la préparation du sorbant modifié par des ions argents dans un procédé comprenant le mélange du sorbant, d'une source de cations argent et d'un solvant choisi dans le groupe comportant un ou plusieurs alcools en C_{1 à 4}, l'eau et un mélange d'eau et d'un solvant miscible dans l'eau, et le séchage.

21. Procédé selon l'une quelconque des revendications 1 à 20, dans lequel le cyano gel de silice comprend le gel de silice lié par covalence sur sa surface à un groupe cyano alkyle.

22. Procédé selon la revendication 20, dans lequel le solvant est un alcool en C_{1 à 4}, et la source de cations argent et l'alcool en C_{1 à 4} sont combinés pour former une solution, et le sorbant est ensuite ajouté pour former une suspension, et de préférence où l'alcool en C_{1 à 4} est le méthanol.

23. Procédé selon la revendication 21, dans lequel le solvant est un alcool en C_{1 à 4}, et la source d'ion argent et l'alcool en C_{1 à 4} Sont chauffés à environ la température de reflux de solvant pour obtenir une solution.

24. Procédé selon la revendication 20, dans lequel le solvant est l'eau ou un mélange d'eau et d'un solvant miscible dans l'eau, le sorbant est un gel de silice en phase inverse, la source d'ion argent est dissoute à température ambiante, et, avant séchage, une solution d'ammoniac est ajoutée à la solution de solvant et de source de cations argent.

25. Procédé selon la revendication 20, dans lequel la source de cations argent est une solution d'un sel d'argent ou d'un complexe d'argent, et de préférence où la source de cations argent comprend au moins un des composés du nitrate d'argent ou de l'acétate d'argent.

26. Procédé selon la revendication 20, dans lequel la source de cations argent est choisie dans le groupe comprenant le nitrate d'argent, l'acétate d'argent, le sulfate d'argent, un complexe de cyano argent et un complexe de thiosulfate d'argent.

27. Procédé selon la revendication 25, dans lequel la source de cations argent comprend un complexe de cyano argent.

28. Procédé selon la revendication 20, dans lequel le sorbant modifié par des ions argents, après séchage, comprend environ 0,1 à environ 15% m/m de sel d'argent.

29. Procédé selon l'une quelconque des revendications 1 à 28, dans lequel le tacrolimus séparé représente au moins environ 93 pour cent de surface par HPLC de tacrolimus.

30. Procédé selon l'une quelconque des revendications 1 à 28, dans lequel le tacrolimus séparé représente moins d'environ 0,20 pour cent de surface par HPLC d'ascomycine.

31. Procédé selon l'une quelconque des revendications 1 à 28, dans lequel le tacrolimus séparé représente moins d'environ 0,04 pour cent de surface par HPLC de dihydrotacrolimus.

32. Procédé selon l'une quelconque des revendications 1 à 28, comprenant en outre la séparation de n'importe quels ions argents élués avec le tacrolimus du tacrolimus.

33. Procédé selon l'une quelconque des revendications 1 à 32, comprenant l'évaporation de l'éluant pour obtenir un résidu, la dissolution du résidu dans un solvant organique, le mélange de la solution résultante avec une solution comprenant un réactif qui précipite les ions argent et la filtration du sel d'argent précipité, où le solvant organique est de préférence choisi dans un groupe comprenant l'acétate d'éthyle, le toluène, l'acétate d'isopropyle ou normale, l'acétate de n-butyle, l'acétate d'isobutyle, l'acétone, l'éthyl méthyl cétone, l'isobutyl méthyl cétone, l'éthanol, le propanol, le n-butanol, l'isobutanol, des mélanges d'acétate d'éthyle et d'acétone, des mélanges de toluène et d'acétone, et leurs mélanges avec de l'eau, et de manière davantage préférée où le solvant organique est l'acétate d'éthyle.

34. Procédé selon la revendication 33, dans lequel le réactif comprend un contre ion choisi dans un groupe comprenant l'acétate, le sulfate, le nitrite, le bromate, le salicylate, l'iodate, le chromate, le carbonate, le citrate, le phosphate, le chlorure, le stéarate, le sulfure, le bromure, l'iodure, le cyanure, le benzoate, l'oxalate, le sulfite et le thiocyanate, et de préférence où le réactif de précipitation est NH₄Cl ou le chlorure de sodium.

35. Procédé selon la revendication 33, comprenant en outre l'ajout du réactif de précipitation d'une quantité de 1 à environ 5 équivalents molaires pour un équivalent molaire d'argent.

36. Procédé selon la revendication 33, dans lequel la solution comprend un réactif qui précipite les ions argent dans une solution aqueuse.

37. Procédé selon la revendication 33, comprenant le passage de l'éluat à travers une colonne remplie d'un mélange de gel de silice et d'un agent de précipitation.

38. Procédé selon l'une quelconque des revendications 1 à 37, comprenant en outre la collecte d'au moins une fraction d'effluent ayant le tacrolimus.

39. Procédé selon la revendication 38, comprenant en outre la cristallisation du tacrolimus séparé.
